# EUROPÄISCHE PATENTSCHRIFT

(11) **EP 2 453 982 B1**
(45) Veröffentlichungstag und Bekanntmachung des Hinweises auf die Patenterteilung: **12.08.2015**
(21) Anmeldenummer: 10723155.7
(22) Anmeldetag: 18.06.2010
(51) Int. Cl.: A61N 5/10

(54) **VERFAHREN ZUR BESTRAHLUNG EINES SICH BEWEGENDEN ZIELVOLUMENS SOWIE BESTRAHLUNGSANLAGE**
METHOD FOR IRRADIATING A MOVING TARGET VOLUME, AND IRRADIATION SYSTEM
PROCÉDÉ D'IRRADIATION D'UN VOLUME CIBLE EN MOUVEMENT ET INSTALLATION D'IRRADIATION

(30) Priorität: 15.07.2009 DE 102009033285
(43) Veröffentlichungstag der Anmeldung: 23.05.2012
(73) Patentinhaber: GSI Helmholtzzentrum für Schwerionenforschung GmbH, 64291 Darmstadt (DE)
(72) Erfinder: BERT, Christoph, 63741 Aschaffenburg (DE); RIETZEL, Eike, 64331 Weiterstadt (DE)
(74) Vertreter: Blumbach Zinngrebe
(86) Internationale Anmeldenummer: PCT/EP2010/058595
(87) Internationale Veröffentlichungsnummer: WO 2011/006731

(56) Entgegenhaltungen:
- DE-A1-102007 014 715
- US-A1- 2009 095 921
- BERT CHRISTOPH ET AL: "4D treatment planning for scanned ion beams" RADIATION ONCOLOGY, BIOMED CENTRAL LTD, LO LNKD- DOI:10.1186/1748-717X-2-24, Bd. 2, Nr. 1, 3. Juli 2007 (2007-07-03), Seite 24, XP021030775 ISSN: 1748-717X
- PARODI K ET AL: "First 4D in-beam PET measurement for beam tracking of a moving phantom with a scanned carbon ion beam" NUCLEAR SCIENCE SYMPOSIUM CONFERENCE RECORD, 2008. NSS '08. IEEE (19-25 OCT. 2008), IEEE, PISCATAWAY, NJ, USA, 19. Oktober 2008 (2008-10-19), Seiten 4520-4524, XP031418564 ISBN: 978-1-4244-2714-7
- PHILLIPS M H ET AL: "Effects of respiratory motion on dose uniformity with a charged particle scanning method" PHYSICS IN MEDICINE AND BIOLOGY, TAYLOR AND FRANCIS LTD. LONDON, GB LNKD- DOI:10.1088/0031-9155/37/1/016, Bd. 37, Nr. 1, 1. Januar 1992 (1992-01-01), Seiten 223-233, XP020021933 ISSN: 0031-9155
- Grözinger, Sven Oliver: "Volume conformal irradiation of moving target volumes with scanned ion beams"[Online] 12. Februar 2004 (2004-02-12), XP002595362 Darmstadt Erlangung des Gradeseines Doktors der Naturwissenschaften,Vom Fachbereich Physik TU Darmstadt Gefunden im Internet: URL:http://tuprints.ulb.tu-darmstadt.de/40 7/> [gefunden am 2010-08-05]

## Beschreibung

Die Erfindung betrifft ein Verfahren zur Bestrahlung eines sich bewegenden Zielvolumens in anderen Gegenständen als menschlichen oder tierischen Körpern, oder zur Simulation einer Bestrahlung eines sich bewegenden Zielvolumens bzw. eine Bestrahlungsanlage zum Bestrahlen eines sich bewegenden Zielvolumens.

Ein derartiges Verfahren wird insbesondere eingesetzt bei einer tatsächlichen und/oder simulierten Bestrahlung eines sich bewegenden Zielvolumens zur genauen Dosisdeposition im Zielvolumen.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, finden jedoch auch in nicht-therapeutischen Gebieten Anwendung. Hierzu gehören beispielsweise Forschungsarbeiten, etwa zur Produktentwicklung, im Rahmen der Partikeltherapie, die an nicht-lebenden Phantomen oder Körpern durchgeführt werden, Bestrahlungen von Materialien, etc.

Hierbei werden geladene Partikel wie z.B. Protonen oder Kohlenstoffionen oder andere Ionen auf hohe Energien beschleunigt, zu einem Partikelstrahl geformt und über ein Hochenergiestrahltransportsystem zu einem oder mehreren Bestrahlungsräumen geführt. In dem Bestrahlungsraum wird das zu bestrahlende Zielvolumen mit dem Partikelstrahl bestrahlt.

Es kann dabei vorkommen, dass sich das zu bestrahlende Zielvolumen bewegt. Beispielsweise kann bei der Bestrahlung eines Patienten eine Bewegung des zu bestrahlenden Tumors durch eine Atembewegung verursacht werden. Eine derartige Bewegung kann auch anhand von als Phantomen bezeichneten Modellobjekten für Forschungszwecke nachgebildet werden.

Insbesondere bei Bestrahlungsverfahren, bei denen eine Vielzahl von Bestrahlungsdosen sukzessive an unterschiedlichen Orten im Zielvolumen deponiert werden soll, also bei einem gescannten Partikelstrahl, ist es schwer, eine gewünschte homogene Dosisverteilung im Zielvolumen zu erreichen, wenn sich das Zielvolumen während des Fortgangs der Bestrahlung bewegt. Eine bekannte Möglichkeit, die Bewegung des Zielvolumens zu kompensieren, sind Bestrahlungsverfahren, die unter den Begriffen "Rescanning", "Gating" und "Tracking" bekannt sind.

Unter "Rescanning" wird verstanden, dass der Strahl bei einer Bestrahlungssitzung mehrfach appliziert wird mit proportional weniger Dosis pro Applikation, so dass sich Fehldosierungen der einzelnen Durchläufe ausmitteln. Unter "Gating" wird verstanden, dass der Strahl nur zu festgelegten Fenstern der Bewegung appliziert wird, so dass der Bewegungseinfluss gemindert oder bestenfalls sogar ausgeschlossen wird. Unter "Tracking" wird verstanden, dass der Strahl, mit dem die Bestrahlung des Zielvolumens erfolgt, der Bewegung des Zielvolumens nachgeführt wird. Falls der Strahl ein Partikelstrahl ist, kann dies beispielsweise erreicht werden, indem ein Strahl durch Magnetsysteme derart abgelenkt wird, dass sein Verlauf der Bewegung des Zielvolumens nachgeführt wird. Gegebenenfalls kann der Strahl auch in seiner Energie variiert werden, um die Eindringtiefe des Strahls der Bewegung des Zielvolumens anzupassen.

Aus der Schrift Grözinger SO et al., "Is Rescanning an Alternative to Online Motion Compensation?", GSI Scientific Report 2003, May 2004, ISSN 0174-0814, S. 176, wird das Rescanning-Verfahren zur Bewegungskompensation beschrieben.

Das Dokument DE 10 2007 014 715 A1 zeigt die Bestimmung von Steuerparametern für eine Bestrahlung eines bewegten Zielvolumens in einem Körper.

Es ist die Aufgabe der Erfindung, ein Verfahren und eine Bestrahlungsanlage bereitzustellen, welche auch bei Bestrahlung eines Zielvolumens mit einem Strahl, der im Rescanningverfahren appliziert wird, eine genaue Dosisdeposition ermöglichen. Die Aufgabe wird durch die unabhängigen Ansprüche gelöst. Bevorzugte Ausgestaltungen der Erfindung sind in den abhängigen Ansprüchen angegeben und werden im Folgenden näher erläutert. Die vorangehende und die folgende Beschreibung der einzelnen Merkmale bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein.

Das erfindungsgemäße Verfahren zur Bestrahlung eines sich bewegenden Zielvolumens in anderen Gegenständen als menschlichen oder tierischen Körpern, oder zur Simulation einer Bestrahlung eines sich bewegenden Zielvolumens weist folgende Schritte auf:
- Bestrahlen des Zielvolumens derart, dass das Zielvolumen im Rescanning-Verfahren bestrahlt wird, bei dem ein Strahl mehrfach über das Zielvolumens gescannt wird, und
- Ermitteln einer Dosisbelegung, die in einem von dem Zielvolumen unterschiedlichen Bereich bei der Bestrahlung des Zielvolumens aufgetreten ist, wobei der Bereich einem anderen Bewegungsmuster als das Zielvolumen unterliegt und/oder wobei der Bereich einer anderen bewegungsbedingten Partikelreichweitenänderung als das Zielvolumen unterliegt,
- wobei die Dosisbelegung, die während der Bestrahlung in dem von dem Zielvolumen unterschiedlichen Bereich auftritt, anhand von während der Bestrahlung aufgezeichneten Daten ermittelt wird,
- wobei die während der Bestrahlung aufgezeichneten Daten erste Daten umfassen, welche die Bewegung des Zielvolumens charakterisieren,
- wobei die während der Bestrahlung aufgezeichneten Daten zweite Daten umfassen, welche die Bewegung des von dem Zielvolumen unterschiedlichen Bereichs charakterisieren und/oder dass die während der Bestrahlung aufgezeichneten Daten dritte Daten umfassen, welche die Bewegung einer die Eindringtiefe des Partikelstrahls ändernden Struktur charakterisieren und
- dass eine nachfolgende Bestrahlung des Zielvolumens bzw. eine Bestrahlungsplanung für das Zielvolumen anhand der für den von dem Zielvolumen unterschiedlichen Bereich ermittelten Dosisbelegung modifiziert wird.

Bei einer Bestrahlung, bei der der Strahl im Rescanning-Verfahren appliziert wird, sind die das Rescanning bestimmenden Größen auf die Bewegung des Zielvolumens abgestimmt. Beispielsweise kann die Art, wie das Rescanning durchgeführt wird, also ein schichtweises Rescanning oder ein volumetrisches Rescanning, auf die Bewegung des Zielvolumens abgestimmt sein. Insbesondere können das Rescanning und die Bewegung des Zielvolumens zeitlich derart aufeinander abgestimmt sein, dass z.B. eine Desynchronisation zwischen Bewegung des Zielvolumens und Applikation der einzelnen Rescanning-Durchgänge vorliegt, sodass der Ausmittelungseffekt beim Rescanning zu tragen kommt. Dies kann beispielsweise erreicht werden, indem die meist in etwa zyklische Bewegung des Zielvolumens während der Bestrahlung überwacht wird und die einzelnen Rescandurchgänge zu passenden Zeitpunkten gestartet werden.

Auf diese Weise werden Interferenzeffekte, die dadurch entstehen, dass sich das Zielvolumen während der Bestrahlung bewegt, und die bewirken, dass es bei einem Strahl ohne Rescanning an Stellen im Zielvolumen zu einer Über- bzw. Unterdosierung in Bezug auf die geplante Dosisverteilung kommen kann, weitgehend vermieden, und zwar dadurch, dass sich bei den einzelnen Rescan-Durchgängen andere Interferenzeffekte auftreten, die sich dann in Summe ausmitteln.

Das erfindungsgemäße Verfahren beruht auf der Erkenntnis, dass gerade das Rescanning, das zur Vermeidung derartiger Interferenzeffekte im Zielvolumen eingesetzt wird, allerdings zu einer nicht vorhersehbaren Dosisdeposition führen kann, und zwar in Bereichen, die bei der Bestrahlung des Zielvolumens ebenso mit einer Dosis belastet werden, wie beispielsweise Bereiche im Eintrittskanal des Strahls. Dies tritt z.B. dann auf, wenn dieser Bereich ein anderes Bewegungsmuster als das Zielvolumen aufweist, da dann zwischen dem Rescanning und der Bewegung des Bereichs aber zumindest eine partielle Synchronisation auftreten kann, selbst wenn beispielsweise zwischen dem Rescanning und der Bewegung des Zielvolumens die gewünschte Desynchronisation vorliegt.

Doch selbst wenn sich der vom Zielvolumen unterschiedliche Bereich gleichartig wie das Zielvolumen bewegt, kann der Bereich mit einer nicht vorhersehbaren Dosis belegt werden. Dies geschieht nämlich auch dann, wenn sich durch die Bewegung im Zielobjekt eine Partikelreichweitenänderung ergibt, die für das Zielvolumen anders ist als für den Bereich. Beispielsweise kann in den Eintrittskanal des Strahls eine stationäre Struktur in periodischer Weise zu liegen kommen, beispielsweise eine durch Atembewegung verschiebliche Rippe, welche die Reichweite des Partikelstrahls beeinflusst, und zwar dort, wo der vom Zielvolumen unterschiedliche Bereich liegt. Wenn sich nun das Rescanning weiterhin auf die Bewegung des Zielvolumens abgestimmt ist, kann ein anderer Synchronisationsgrad zwischen dem Zielvolumen und dem Rescanning vorliegen als zwischen dem Bereich und dem Rescanning.

Letztlich rührt diese "Fehldosierung" im weiteren Bereich daher, dass der Bereich und das Zielvolumen ein differentielles Bewegungsmuster aufweisen und/oder dass im Eintrittkanal eine Struktur vorliegen kann, die gegenüber dem Zielvolumen ein differentielles Bewegungsmuster aufweist.

Der Bereich kann z.B. ein weiteres Volumen sein, das ebenso wie das sich bewegende Zielvolumen in dem zu bestrahlenden Objekt im Eintrittskanal liegt, oder das zumindest teilweise innerhalb des Sicherheitssaumes liegt, der üblicherweise bei einem Rescanning-Verfahren um das Zielvolumen gelegt wird. Beispielsweise kann das weitere Volumen distal des Zielvolumens liegen, und zwar dort, wo aufgrund des Sicherheitssaumes beim Rescanning immer auch ein gewisser Dosisanteil außerhalb des Zielvolumens deponiert wird.

Aufgrund der Dosisdeposition in dem vom Zielvolumen unterschiedlichen Bereich kann es zu einer Überdosierung und auch zu einer Schädigung kommen, die im Rahmen der ursprünglichen Bestrahlungsplanung, also bei der Referenzbestrahlungsplanung, nicht abzusehen war. Dies ist für ein als Risikoorgan klassifiziertes Volumen besonders gefährlich, doch auch bei einem nicht als Risikoorgan klassifizierten Volumen kann eine Überdosierung zu Komplikationen führen. Dies betrifft auch Bereiche, die gemäß der ursprünglichen Bestrahlungsplanung nicht mit einer Dosis belegt werden, die aber aufgrund der unterschiedlichen differentiellen Bewegungsmuster dennoch mit einer Dosis belegt werden.

Mit dem erfindungsgemäßen Verfahren kann nun aber die während einer Bestrahlung in dem vom Zielvolumen unterschiedlichen Bereich deponierte Dosis ermittelt werden, sodass die Unsicherheiten bezüglich der Dosisbelastung des Objekts beseitigt werden.

Insbesondere kann die Dosisbelegung die während der Bestrahlung in dem Bereich auftritt, anhand von während der Bestrahlung aufgezeichneten Daten ermittelt wird, z.B. anhand von Daten, die die Anpassung des Rescanning-Verfahrens an die Bewegung des Zielvolumens charakterisieren. Hierzu können z.B. die Werte eines Therapie-Online-Monitors, eines Sensor zur Detektion der Bewegung des Zielvolumens und der weiteren Bereichs lateral, proximal oder distal des Zielvolumens, die Trajektorie eines Bewegungserfassungssystems, die extrahierte Intensität, die Zeitpunkte, zu denen die einzelnen Rescanning-Durchgänge gestartet werden, usw. protokolliert werden.

In einer Ausführungsform kann bei Ermitteln der Dosisbelegung eine Dosisberechnung durchgeführt wird, mit der eine Dosis bestimmt wird, die in dem Bereich deponiert wird, wobei die Dosisberechnung unter Verwendung einer während der Bestrahlung durchgeführten Anpassung des Rescanning-Verfahren an das Zielvolumen erfolgt. Auf diese Weise kann genau festgelegt werden, welche Dosis in dem Bereich bei der Durchführung der Bestrahlung tatsächlich deponiert worden ist.

Die Dosisberechnung kann dabei auf verschiedene Weise durchgeführt werden. Es kann z.B. eine Berechnung der Dosisverteilung für den Bereich durchgeführt werden, die Berechnung eines Dosis-Volumen-Histogramms, die Berechnung der maximalen Dosis, oder ähnliche bekannte Verfahren aus der Strahlentherapie, die eingesetzt werden, um eine Dosisbelastung für einen Bereich anzugeben.

In einer Ausführungsform können die während der Bestrahlung aufgezeichneten Daten erste Daten umfassen, welche die Bewegung des Zielvolumens charakterisieren. Alternativ oder zusätzlich können die Daten zweite Daten umfassen, welche die Bewegung des vom Zielvolumen unterschiedlichen Bereichs charakterisieren. Alternativ oder zusätzlich können die Daten dritte Daten umfassen, welche die Bewegung einer die Eindringtiefe des Partikelstrahls ändernden Struktur charakterisieren. All diese Informationen eignen sich, ein differentielles Bewegungsmuster zu detektieren, das wie oben beschrieben zu einer im Rahmen der Bestrahlungsplanung nicht vorhersehbaren Dosisdeposition im vom Zielvolumen unterschiedlichen Bereich führt.

Daten, welche die Bewegung charakterisieren, können z.B. Bilddaten sein, in denen die Bewegung der beobachteten Strukturen abgebildet ist, z.B. mittels Fluoroskopie aufgezeichnet, oder aber auch Bewegungsdaten, welche indirekte Rückschlüsse auf die Bewegung der Struktur zulassen, wie beispielsweise eine EKG oder ein Atemsensor.

In einer vorteilhaften Ausführungsform wird eine nachfolgende Bestrahlung oder eine Bestrahlungsplanung des Zielvolumens anhand der ermittelten Dosisbelegung modifiziert.

Dies kann dadurch erfolgen, dass insbesondere bei einer fraktionierten Bestrahlung die Bestrahlungsplanung modifiziert oder neu durchgeführt und an die Dosis angepasst wird, mit der der Bereich bereits belastet worden ist. Es kann aber auch ein Bestrahlungsverfahren, d.h. eine Bestrahlungsart oder Bestrahlungstechnik, geändert werden. Wenn es beispielsweise zu einer großen Dosisbelastung in dem Bereich gekommen ist, kann z.B. festgelegt werden, für die nachfolgenden Bestrahlungen ein Gating-Verfahren zu verwenden anstelle des Rescanning-Verfahrens.

In einer vorteilhaften Ausführungsform kann die Dosisbelegung des Bereichs auch während der Bestrahlung ermittelt werden. Im Falle von zu großen Abweichungen von geplanten Werten kann dann die Bestrahlung unterbrochen oder abgebrochen (Interlock).

Unter Verfahren zur Bestrahlung werden hier sowohl Verfahren verstanden, bei denen tatsächlich eine Bestrahlung eines Zielvolumens durchgeführt wird, als auch Verfahren, bei denen eine Bestrahlung eines Zielvolumens lediglich simuliert wird. Bei einer simulierten Bestrahlung wird der Strahl einer "virtuellen", also simulierten, Bewegung des Zielvolumens nachgeführt.

Derartige Simulationen werden oftmals im Rahmen einer Bestrahlungsplanung eingesetzt und liefern Informationen, ob eine Bestrahlung den gewünschten Kriterien entspricht oder ob eine Bestrahlung geändert werden soll. Beispielsweise können Bestrahlungen für eine Vielzahl von unterschiedlichen Bewegungsmustern des Zielvolumens simuliert werden. Bei jeder dieser simulierten Bestrahlungen kann die Dosisbelegung ermittelt werden, die in dem vom Zielvolumen unterschiedlichen Bereich deponiert würde und die sich aufgrund des Bewegungsmusters des Zielvolumens und der sich damit zusammenhängenden Steuerung des Rescanning-Verfahrens ergeben würde.

Wird diese Simulation bei einer Vielzahl von unterschiedlichen Bewegungsmustern des Zielvolumens durchgeführt, ohne dass unerwünschte oder gar gefährliche Überdosierungen in dem vom Zielvolumen unterschiedlichen Bereich auftreten, kann angenommen werden, dass auch bei einer tatsächlich durchgeführten Bestrahlung keine Überdosierungen auftreten. Auf diese Weise kann beispielsweise simuliert werden, ob es durch das geplante Rescanning zu einer gefährlichen Überdosierung in einer Risikostruktur kommen könnte.

Auch für den Fall, dass die Dosisbelegung in dem Bereich für eine Vielzahl von Bewegungsmustern des Zielvolumens simuliert worden ist, kann eine Überprüfung der tatsächlich applizierten Dosisverteilung in dem Bereich trotzdem sinnvoll sein, falls tatsächlich eine Bestrahlung durchgeführt wird.

Das Verfahren kann also einerseits bei therapeutischen Behandlungen eingesetzt werden, bei denen eine tatsächliche Bestrahlung eines menschlichen oder tierischen Körpers stattfindet. Das Verfahren kann aber andererseits und erfindungsgemäss als nichttherapeutisches Verfahren eingesetzt werden, beispielsweise indem eine Bestrahlung lediglich simuliert wird oder in dem eine Bestrahlung von anderen Gegenständen als einen menschlichen oder tierischen Körper durchgeführt wird, wie z.B. die Bestrahlung eines Phantoms oder die Bestrahlung von Materialien im Allgemeinen.

Die erfindungsgemäße Bestrahlungsanlage zum Bestrahlen eines sich bewegenden Zielvolumens weist auf:
- eine Bestrahlungsvorrichtung, die ausgebildet ist zum Bestrahlen des Zielvolumens derart, dass bei einer Bestrahlung das Zielvolumen im Rescanning-Verfahren bestrahlbar ist, bei dem ein Strahl mehrfach über das Zielvolumens gescannt wird, und
- eine Überwachungsvorrichtung, die ausgebildet ist zum Ermitteln einer Dosisbelegung, die in einem von dem Zielvolumen unterschiedlichen Bereich bei der Bestrahlung des Zielvolumens auftritt, wobei der Bereich dadurch charakterisiert ist, dass der Bereich einem anderen Bewegungsmuster und/oder einer anderen bewegungsbedingten Partikelreichweitenänderung als das Zielvolumen unterliegt,
- eine Bewegungsüberwachungsvorrichtung zum Aufzeichnen von ersten Daten während der Bestrahlung, welche die Bewegung des Zielvolumens charakterisieren,
- wobei der Bereich dadurch charakterisiert ist, dass der Bereich einem anderen Bewegungsmuster und/oder einer anderen bewegungsbedingten Partikelreichweitenänderung als das Zielvolumen unterliegt und
- wobei die Überwachungsvorrichtung dazu ausgebildet ist, die Dosisbelegung in dem unterschiedlichen Bereich anhand der aufgezeichneten Daten der Bewegungsüberwachungsvorrichtung zu ermitteln,
- wobei die Bewegungsüberwachungsvorrichtung während der Bestrahlung zweite Daten aufzeichnet, welche die Bewegung des von dem Zielvolumen unterschiedlichen Bereichs charakterisieren und/oder wobei die Bewegungsüberwachungsvorrichtung während der Bestrahlung dritte Daten aufzeichnet, welche die Bewegung einer die Eindringtiefe des Partikelstrahls ändernden Struktur charakterisieren,
- wobei aus den ersten und zweiten oder den ersten und dritten Daten eine während der Bestrahlung durchgeführte Anpassung des Rescanning-Verfahren an die Bewegung des von dem Zielvolumen unterschiedlichen Bereichs ermittelbar ist;
- Ferner mit einer Bestrahlungssteuerungsvorrichtung, die ausgebildet ist zum Modifizieren einer nachfolgenden Bestrahlung des Zielvolumens unter Verwendung der für den von dem Zielvolumen:unterschiedlichen Bereich ermittelten Dosisbelegung.

Die Bestrahlungsanlage kann zusätzlich eine Erfassungseinrichtung aufweisen zum Aufzeichnen von ersten Daten, aus denen eine während der Bestrahlung durchgeführte Anpassung des Rescanning-Verfahren an die Bewegung des Zielvolumens ermittelbar ist.

Die Überwachungsvorrichtung kann ausgebildet sein zum Durchführen einer Dosisberechnung, mit der eine Dosis bestimmt wird, die bei der Bestrahlung in dem Bereich deponiert wird, und wobei die Dosisberechnung unter Verwendung der ersten Daten erfolgt.

Die Bestrahlungsanlage kann ferner eine Bewegungsüberwachungsvorrichtung aufweisen, um während der Bestrahlung Daten aufzuzeichnen, welche eine Bewegung des Zielvolumens, des Bereichs und/oder einer die Eindringtiefe des Partikelstrahls ändernden Struktur charakterisieren. Die Überwachungsvorrichtung kann in diesem Fall dazu ausgebildet sein, die Dosisbelegung in dem unterschiedlichen Bereich anhand der aufgezeichneten Daten zu ermitteln.

Weiterhin kann die Bestrahlungsanlage eine Bestrahlungsteuerungsvorrichtung umfassen, die ausgebildet ist zum Modifizieren einer nachfolgenden Bestrahlung des Zielvolumens oder eine Änderung einer Bestrahlungsplanung für das Zielvolumen unter Verwendung der ermittelten Dosisbelegung, insbesondere durch eine Änderung der Bestrahlungsplanung und/oder durch einen Wechsel des Bestrahlungsverfahrens.

Ausführungsformen der Erfindung sowie vorteilhafte Weiterbildungen gemäß den Merkmalen der abhängigen Ansprüche werden anhand der folgenden Zeichnung näher erläutert, ohne jedoch darauf beschränkt zu sein. Hierbei sind in allen Figuren gleiche Gegenstände mit gleichen Bezugszeichen versehen.
Fig. 1 zeigt eine Bestrahlungsanlage gemäß einer Ausführungsform der Erfindung;
Fig. 2 zeigt eine Darstellung eines Ausschnittes der Bestrahlungsanlage, anhand der eine Ausführung des erfindungsgemäßen Verfahrens erläutert wird;
Fig. 3 eine zu Fig. 2 ähnliche Darstellung, anhand der eine weitere Ausführung des erfindungsgemäßen Verfahrens erläutert wird;
Fig. 4 zeigt ein schematisches Ablaufdiagramm einer Ausführungsform des erfindungsgemäßen Verfahrens.

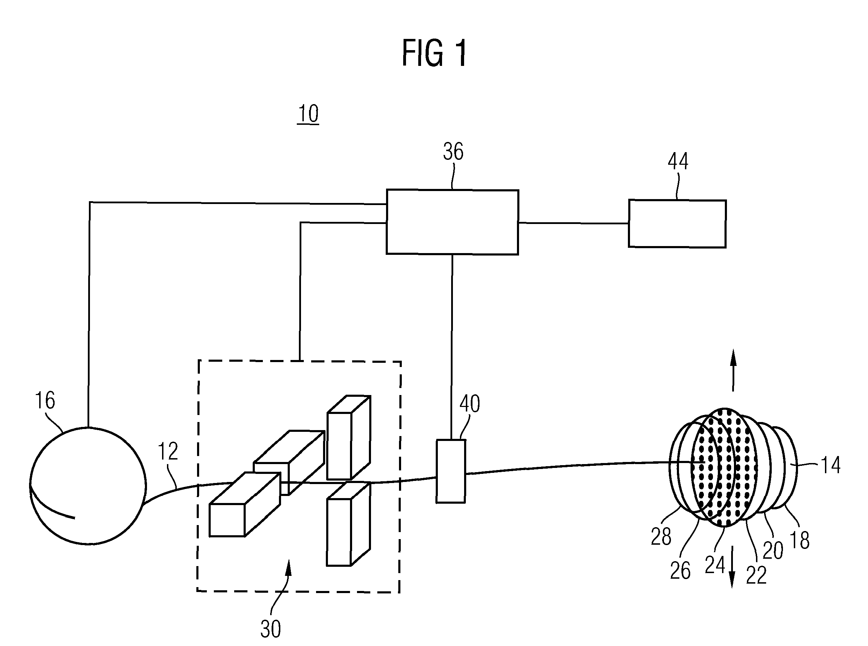

Figur 1 zeigt in stark schematisierter Darstellung einen Aufbau einer als Partikeltherapieanlage 10 aufgebauten Bestrahlungsanlage. Die Partikeltherapieanlage 10 wird zur Bestrahlung eines auf einer Positioniervorrichtung angeordneten Körpers mit einem Strahl aus Partikeln, der im Folgenden als Partikelstrahl 12 bezeichnet ist, verwendet. Insbesondere kann ein tumorerkranktes Gewebe 14 eines Patienten mit dem Partikelstrahl 12 bestrahlt werden. Es ist ebenfalls vorgesehen, die Partikelstrahlanlage 10 zur Bestrahlung eines nichtlebenden Körpers, insbesondere eines Wasserphantoms oder anderen Phantomen einzusetzen. Die Bestrahlung des Wasserphantoms kann beispielsweise zu Zwecken der Überprüfung und Verifizierung von Bestrahlungsparametern vor und/oder nach einer erfolgten Bestrahlung eines Patienten erfolgen. Es ist aber auch vorgesehen, andere Körper, insbesondere Versuchsaufbauten wie beispielsweise Zellkulturen oder Bakterienkulturen zu Forschungszwecken mit dem Partikelstrahl 12 zu bestrahlen. In allen Fällen kann es sich um bewegte oder ruhende Körper handeln.

Die Partikeltherapieanlage 10 weist typischerweise eine Beschleunigereinheit 16 auf, z.B. ein Synchrotron, ein Zyklotron oder einen sonstigen Beschleuniger, der einen Partikelstrahl 12 mit der zur Bestrahlung notwendigen Energie bereitstellt. Als Partikel werden vornehmlich Teilchen wie beispielsweise Protonen, Pionen, Heliumionen, Kohlenstoffionen oder Ionen anderer Elemente eingesetzt. Typischerweise hat einen Partikelstrahl 12 einen Strahldurchmesser von 3-10 mm.

In dem zu bestrahlenden Zielvolumen 14 sind Schichten 18, 20, 22, 24, 26 und 28 angedeutet, welche isoenergetischen Schichten entsprechen. Eine isoenergetische Schicht 18, 20, 22, 24, 26 oder 28 entspricht jeweils Eindringtiefe des Braggpeaks für eine bestimmte Energie des Partikelstrahls 12.

Als Scanverfahren wird bevorzugt ein Rasterscan-Verfahren verwendet, bei dem ein Partikelstrahl von Rasterpunkt zu Rasterpunkt geführt wird ohne zwangsläufige Abschaltung bei einem Übergang von einem Rasterpunkt zum nächsten. Es können auch Spotscan-Verfahren mit Abschaltung des Partikelstrahls zwischen den einzelnen Rasterpunkten oder andere Scan-Verfahren wie beispielsweise kontinuierliche Scanverfahren eingesetzt werden. In Fig. 1 ist das Scan-Verfahren schematisch illustriert, anhand einiger schwarzer Punkte, welche in einem schichtweise aufgebauten Zielvolumen 14 dargestellt sind und welche sukzessive mit dem Partikelstrahl 12 angefahren werden.

Der Partikelstrahl 12 wird in seiner lateralen Auslenkung mithilfe von Scan-Magneten 30 beeinflusst, d.h. in seiner Position senkrecht zu Strahlverlaufsrichtung, auch als x- und y-Richtung bezeichnet, abgelenkt.

Ein Rescanning kann schichtweise durchgeführt werden, so dass die Dosis einer Schicht 18, 20, 22, 24, 26 und 28 aufgeteilt und die Schicht in sukzessiven Applikationen bzw. Rescanning-Durchgängen mehrfach bestrahlt wird, oder aber auch mit einem volumetrischen Rescanning, bei dem sich ein Rescan-Durchgang über mehrere Schichten 18, 20, 22, 24, 26 und 28 erstreckt. Beim volumetrischen Rescanning, also bei einem Rescanning, das zumindest teilweise in Verlaufsrichtung des Partikelstrahls durchgeführt wird, sind üblicherweise schnelle Energiewechsel notwendig. Hierzu kann eine Energiemodulationsvorrichtung im Strahlgang angeordnet werden, z.B. ein System aus beweglichen Keilen.

Die Bestrahlungsanlage 10 weist ferner eine Ablaufsteuerung 36 auf sowie Detektoren zur Überwachung der Strahlparameter 40. Die Anordnung der hier gezeigten Komponenten der Partikelstrahlanlage ist lediglich beispielhaft. Auch Anordnungen in anderer Reihenfolge sind denkbar.

Die Ablaufsteuerung 36, also das Kontrollsystem der Anlage, steuert die einzelnen Komponenten der Anlage, wie beispielsweise den Beschleuniger 16 und die Scan-Magnete 30 und sammelt Messdaten wie beispielsweise die Daten der Detektoren zur Überwachung der Strahlparameter 40. Üblicherweise erfolgt die Steuerung aufgrund eines Bestrahlungsplans, der von einer Bestrahlungsplanungseinrichtung 44 bereitgestellt wird.

Der hier gezeigte Aufbau ist beispielhaft für einen möglichen Aufbau einer Partikeltherapieanlage 10. In einer derartigen Partikeltherapieanlage können Ausführungsformen der Erfindung implementiert werden. Mögliche Ausführungsformen werden anhand der nachfolgenden Zeichnung näher erläutert.

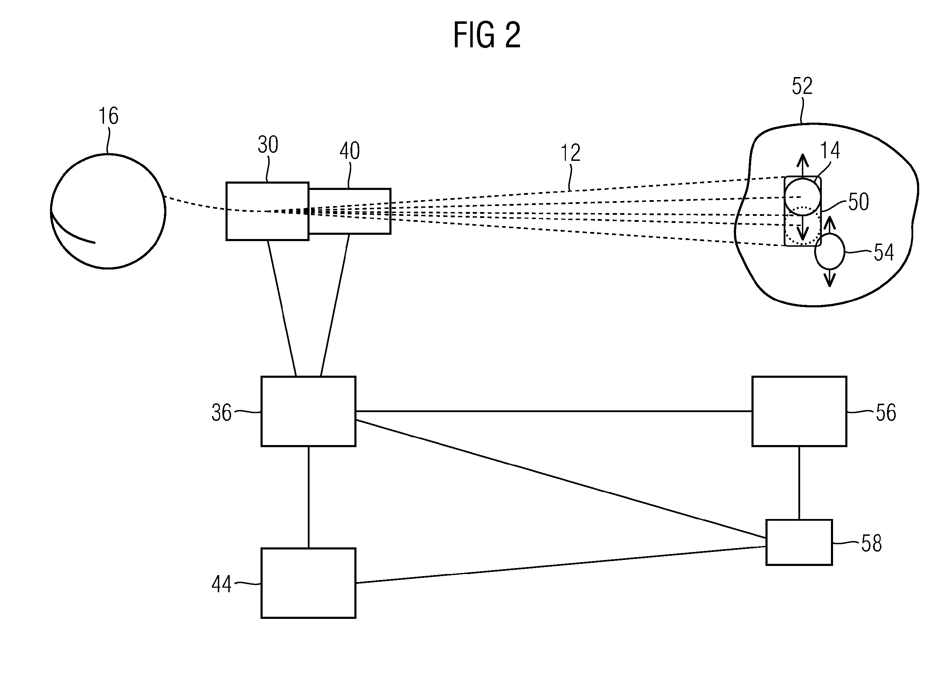

Fig. 2 zeigt in schematischer Darstellung ein zu bestrahlendes Zielobjekt 52, in dem sich das zu bestrahlende Zielvolumen 14 befindet. Ferner ist ein weiterer Bereich 54 gezeigt, der sich in dem zu bestrahlenden Zielobjekt 52 befindet, und er beispielsweise ein zu schonendes Risikoorganen darstellen kann. Das Zielvolumen 14 kann beispielsweise ein hinter einem Sternum gelegener Lungentumor sein, der weitere Bereich 54 das dahinter im Mediastinum gelegene Herz oder ein weiteres wichtiges Bereiche des Mediastinum.

Um das Zielvolumen 14 zu bestrahlen, wird ein größerer Bereich 50 bestrahlt, der dem Bewegungsumfang des Zielvolumens 14 angepasst ist - ein Lungentumor kann sich beispielsweise um 15 mm atmungsbedingt bewegen. Dieser größere Bereich 50 deckt das Zielvolumen 14 mitsamt Sicherheitssaum in allen Bewegungszuständen ab und wird im Rescanning-Verfahren bestrahlt, beispielsweise kann die in diesem größeren Bereich 50 zu deponierende Dosis auf 15 Rescanning-Durchgänge aufgeteilt werden.

Durch diese Bestrahlung des Zielvolumens 14 wird auch der weitere Bereich 54 zumindest teilweise mit einer Dosis belastet. Das Zielvolumen 14 bewegt sich dabei während der Bestrahlung, angedeutet durch die Pfeile. Die Bestrahlung im Rescanning-Verfahren ist dabei auf die Bewegung des Zielvolumens 14 abgestimmt. Dies kann beispielsweise dadurch geschehen, dass darauf geachtet wird, dass die einzelnen Rescan-Durchgänge nicht synchron mit der Bewegung des Zielvolumens 14 ausgeführt werden, um den gewünschten Mittelungseffekt zu erreichen.

Um dies zu erreichen, ist eine Bewegungsdetektionsvorrichtung 56 vorgesehen, z.B. ein externer Sensor oder ein Fluoroskopiesystem, mit der die während einer Bestrahlung auftretende Bewegung des Zielvolumens 14 detektiert werden kann. Mithilfe der so erzeugten Messdaten kann die Ablaufsteuerung 36 den Partikelstrahl 12 entsprechend steuern, so dass der Partikelstrahl 12 im Rescanning-Verfahren desynchronisiert wird. Je nach Regelmäßigkeit der Bewegung kann allerdings auch die Desynchronisation durch entsprechende Planung erreicht werden, ohne dass die Bewegung des Zielvolumens eigens während der Bestrahlung vollständig überwacht wird.

Der weitere Bereich 54, z.B. das Herz, bewegt sich allerdings mit anderem Rhythmus und weist damit ein anderes Bewegungsmuster als das Zielvolumen 14 auf. Daher kann es vorkommen, dass die Applikation der Rescanning-Durchgänge und die Bewegung des weiteren Bereichs 54 ein höheres Maß an Synchronisierung aufweisen, sodass es zu einer ungewollt hohen Dosisbelegung des weiteren Bereichs 54 kommt, die von einer ursprünglichen Planung abweicht und/oder die nicht mehr tolerierbar ist. Die Bewegung des weiteren Bereichs 54 kann ebenfalls von der Bewegungsdetektionsvorrichtung 56 erfasst werden.

Als Überwachungsvorrichtung 58, mit der diese Dosisbelegung detektiert werden kann, ist eine Rechnereinheit vorgesehen. Diese Rechnereinheit bekommt als Eingangsdaten die Messdaten der Bewegungsdetektionsvorrichtung 56 und/oder Daten, welche die Steuerung des Rescanning-Verfahrens beschreiben, z.B. Steuerdaten, die die Startzeitpunkte der einzelnen Rescanning-Durchgänge angeben und welche von der Ablaufsteuerung 36 stammen. Aus diesen Daten und aus der ursprünglichen Bestrahlungsplanung kann die Überwachungsvorrichtung 58 die Dosisbelegung ermitteln, mit der der weitere Bereich 54 bei der Bestrahlung tatsächlich belegt worden ist.

Diese ermittelte Dosisbelegung kann dann wiederum der Ablaufsteuerung 36 zur Verfügung gestellt werden, welche hierauf basierend den Bestrahlungsverlauf modifizieren kann, oder aber auch einer Bestrahlungsplanungsvorrichtung 44, welche einen bestehenden Bestrahlungsplan für nachfolgende Sitzungen modifizieren kann.

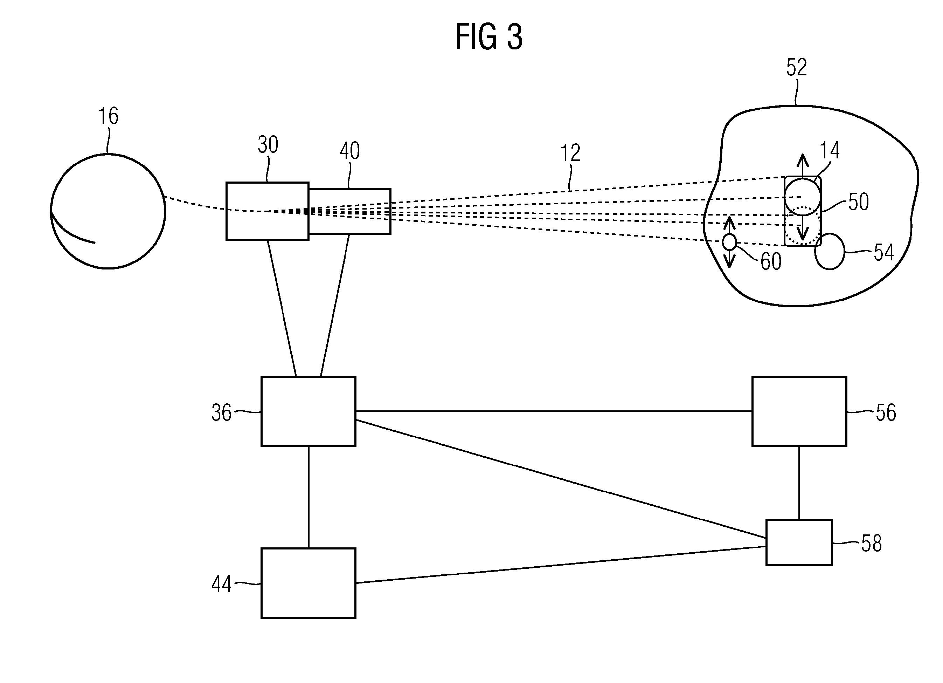

Fig. 3 unterscheidet sich von Fig. 2 insofern, als dass eine leicht geänderter Aufbau, z.B. bedingt durch andere anatomische Verhältnisse, im Zielobjekt 52 vorliegt. Der weitere Bereich 54 bewegt sich in diesem Falle nicht, während im Eintrittskanal eine Struktur 60 liegt, die die Reichweite des Partikelstrahls 12 beeinflusst und die ein anderes Bewegungsmuster aufweist als das zu bestrahlende Zielvolumen 14. Die Struktur 60 kann beispielsweise ein Knochen sein, wie z.B. eine Rippe.

Wenn die Struktur 60 im Eintrittskanal ein anderes Bewegungsmuster aufweist als das Zielvolumen 14, kann es dazukommen, dass es im weiteren Bereich 54 zu einer ungewollten Dosisüberhöhung kommt. Dies kann beispielsweise dann auftreten, wenn sich die Struktur 60 weitgehend synchron mit der Applikation der Rescanning-Durchgänge bewegt, sodass sich die Reichweite des Partikelstrahls 12 ebenfalls synchron mit den Rescanning-Durchgängen ändert und deswegen vermehrt in gleicher Tiefe den weiteren Bereich 54 belastet.

Die Dosis, mit der der weitere Bereich 54 belegt wird, kann bei ausreichender Rechenleistung bereits während der Strahlapplikation zumindest teilweise berechnet werden, und - falls eine intolerable Dosisüberhöhung festgestellt wird - kann die Bestrahlung durch einen Interlock abgebrochen werden.

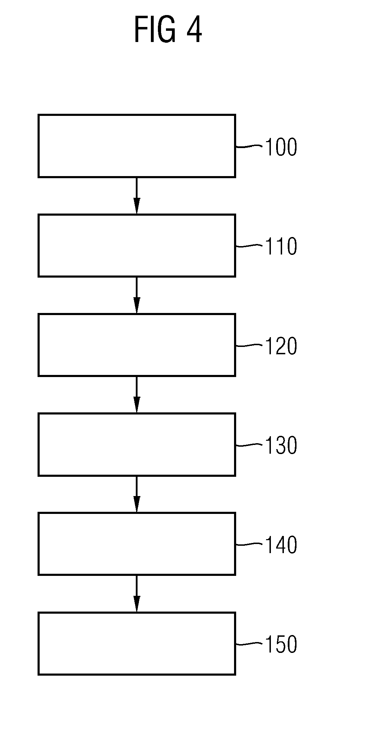

Fig. 4 zeigt schematisch Verfahrensschritte, die in einer möglichen Ausführungsform des Verfahrens durchgeführt werden können.

In einem ersten Schritt (Schritt 100) wird das bewegte Zielvolumen in einem Rescanning-Verfahren bestrahlt. Während der Bestrahlung wird das Rescanning der Bewegung des Zielvolumens angepasst(Schritt 110). Hierzu kann die Bewegung des Zielvolumens und eventuell zusätzlich die Bewegung des weiteren Bereichs oder von weiteren im Zielobjekt vorhandenen, die Reichweite des Partikelstrahls beeinflussenden Strukturen überwacht werden (Schritt 120). Anhand der zeitlichen Charakteristik des Rescanning-Verfahrens, die bei der tatsächlich durchgeführten Bestrahlung auftritt, wird eine Dosisbelegung ermittelt (Schritt 130), welche in einem Bereich aufgetreten ist, der ein unterschiedliches Bewegungsmuster als das Zielvolumen aufweist. Kann die ermittelte Dosisbelegung toleriert werden, so kann die Bestrahlung wie geplant weiter fortgeführt werden (Schritt 140), entweder wie geplant während der gleichen Bestrahlungssitzung oder wie geplant bei einer folgenden Bestrahlungssitzung. Andernfalls kann die Bestrahlung des Zielvolumens oder eine Bestrahlungsplanung für das Zielvolumen geändert werden (Schritt 150).

Alle im Zusammenhang mit den Figuren gezeigten Ausführungsformen, welche anhand einer tatsächlichen Bestrahlung erklärt und beschrieben wurden, können ebenso lediglich simuliert werden, also im Rahmen einer simulierten Bestrahlung angewendet werden.

### Bezugszeichenliste

- 10: Partikeltherapieanlage
- 12: Partikelstrahl
- 14: Zielvolumen
- 16: Beschleunigereinheit
- 18 - 28: Schichten
- 30: Scan-Magnete
- 36: Ablaufsteuerung
- 40: Detektoren zur Überwachung der Strahlparameter
- 44: Bestrahlungsplanungsseinrichtung

- 50: größerer Bereich
- 52: Zielobjekt
- 54: weiterer Bereich
- 56: Bewegungsdetektionsvorrichtung
- 58: Überwachungsvorrichtung
- 60: Struktur

- 100: Schritt 100
- 110: Schritt 110
- 120: Schritt 120
- 130: Schritt 130
- 140: Schritt 140
- 150: Schritt 150

## Patentansprüche

1. Verfahren zur Bestrahlung eines sich bewegenden Zielvolumens (14) in anderen Gegenständen als menschlichen oder tierischen Körpern, oder zur Simulation einer Bestrahlung eines sich bewegenden Zielvolumens mit folgenden Schritten:
- Bestrahlen des Zielvolumens (14) derart, dass das Zielvolumen (14) im Rescanning-Verfahren bestrahlt wird, bei dem ein Strahl mehrfach über das Zielvolumen (14) gescannt wird, und
- Ermitteln einer Dosisbelegung, die in einem von dem Zielvolumen (14) unterschiedlichen Bereich (40, 54) bei der Bestrahlung des Zielvolumens (14) aufgetreten ist, wobei der Bereich (40, 54,) einem anderen Bewegungsmuster als das Zielvolumen (14) unterliegt und/oder wobei der Bereich (40, 54) einer anderen bewegungsbedingten Partikelreichweitenänderung als das Zielvolumen unterliegt,
wobei die Dosisbelegung, die während der Bestrahlung in dem von dem Zielvolumen unterschiedlichen Bereich (40, 54) auftritt, anhand von während der Bestrahlung aufgezeichneten Daten ermittelt wird,
wobei die während der Bestrahlung aufgezeichneten Daten erste Daten umfassen, welche die Bewegung des Zielvolumens charakterisieren,
**gekennzeichnet durch** die Schritte,
dass die während der Bestrahlung aufgezeichneten Daten zweite Daten umfassen, welche die Bewegung des von dem Zielvolumen unterschiedlichen Bereichs (40, 54) charakterisieren und/oder dass die während der Bestrahlung aufgezeichneten Daten dritte Daten umfassen, welche die Bewegung einer die Eindringtiefe des Partikelstrahls ändernden Struktur charakterisieren und
dass eine nachfolgende Bestrahlung des Zielvolumens (14) bzw. eine Bestrahlungsplanung für das Zielvolumen (14) anhand der für den von dem Zielvolumen unterschiedlichen Bereich (40, 54) ermittelten Dosisbelegung modifiziert wird.

2. Verfahren nach Anspruch 1, wobei beim Ermitteln der Dosisbelegung eine Dosisberechnung durchgeführt wird, mit der eine Dosis bestimmt wird, die in dem Bereich (40, 54) deponiert wird, wobei die Dosisberechnung unter Verwendung einer während der Bestrahlung durchgeführten Anpassung des Rescanning-Verfahren an das Zielvolumen erfolgt.

3. Verfahren nach einem der Ansprüche 1 oder 2, wobei die nachfolgende Bestrahlung durch eine Änderung der Bestrahlungsplanung und/oder durch einen Wechsel eines Bestrahlungsverfahrens modifiziert wird.

4. Bestrahlungsanlage zum Bestrahlen eines sich bewegenden Zielvolumens (14)
- mit einer Bestrahlungsvorrichtung (10), die ausgebildet ist zum Bestrahlen des Zielvolumens (14) derart, dass bei einer Bestrahlung das Zielvolumen (14) im Rescanning-Verfahren bestrahlbar ist, bei dem ein Strahl mehrfach über das Zielvolumens (14) gescannt wird,
- mit einer Überwachungsvorrichtung (58), die ausgebildet ist zum Ermitteln einer Dosisbelegung, die in einem von dem Zielvolumen (14) unterschiedlichen Bereich (40, 54) bei der Bestrahlung des Zielvolumens (14) auftritt und
- mit einer Bewegungsüberwachungsvorrichtung zum Aufzeichnen von ersten Daten während der Bestrahlung, welche die Bewegung des Zielvolumens charakterisieren,
- wobei der Bereich (40, 54) dadurch charakterisiert ist, dass der Bereich einem anderen Bewegungsmuster und/oder einer anderen bewegungsbedingten Partikelreichweitenänderung als das Zielvolumen (14) unterliegt und
- wobei die Überwachungsvorrichtung dazu ausgebildet ist, die Dosisbelegung in dem unterschiedlichen Bereich anhand der aufgezeichneten Daten der Bewegungsüberwachungsvorrichtung zu ermitteln,
**gekennzeichnet dadurch**
- **dass** die Bewegungsüberwachungsvorrichtung während der Bestrahlung zweite Daten aufzeichnet, welche die Bewegung des von dem Zielvolumen unterschiedlichen Bereichs (40, 54) charakterisieren und/oder dass die Bewegungsüberwachungsvorrichtung während der Bestrahlung dritte Daten aufzeichnet, welche die Bewegung einer die Eindringtiefe des Partikelstrahls ändernden Struktur charakterisieren,
- wobei aus den ersten und zweiten oder den ersten und dritten Daten eine während der Bestrahlung durchgeführte Anpassung des Rescanning-Verfahren an die Bewegung des von dem Zielvolumen unterschiedlichen Bereichs ermittelbar ist;
- Ferner mit einer Bestrahlungssteuerungsvorrichtung (36, 44), die ausgebildet ist zum Modifizieren einer nachfolgenden Bestrahlung des Zielvolumens (14) unter Verwendung der für den von dem Zielvolumen unterschiedlichen Bereich (40, 54) ermittelten Dosisbelegung.

5. Bestrahlungsanlage nach Anspruch 4, wobei die Bestrahlungsanlage eine Erfassungseinrichtung aufweist, die aus den ersten Daten eine während der Bestrahlung durchgeführte Anpassung des Rescanning-Verfahren an die Bewegung des Zielvolumens ermittelt.

6. Bestrahlungsanlage nach Anspruch 5, wobei
die Überwachungsvorrichtung (58) ausgebildet ist zum Durchführen einer Dosisberechnung, mit der eine Dosis bestimmt wird, die bei der Bestrahlung in dem Bereich (40, 54) deponiert wird, und wobei die Dosisberechnung unter Verwendung der ersten Daten erfolgt.

7. Bestrahlungsanlage nach einem der Ansprüche 4 bis 6, wobei das Modifizieren der nachfolgenden Bestrahlung des Zielvolumens (14) durch eine Änderung der Bestrahlungsplanung und/oder durch einen Wechsel des Bestrahlungsverfahrens erfolgt.

## Claims

1. A method for irradiating a moving target volume (14) in objects other than human or animal bodies, or for simulating irradiation of a moving target volume, comprising:
- irradiating the target volume (14) such that the target volume (14) is irradiated in a rescanning process in which a beam is repeatedly scanned over the target volume (14); and
- determining a dose distribution that occurred in a region (40, 54) different from the target volume (14) during the irradiation of the target volume (14), wherein said region (40, 54) is subject to a different movement pattern than the target volume (14) and/or wherein said region (40, 54) is subject to a different motion-related change in particle range than the target volume,
wherein the dose distribution occurring during the irradiation in the region (40, 54) different from the target volume is determined based on data recorded during the irradiation;
wherein the data recorded during the irradiation comprise first data which are representative for the movement of the target volume;
**characterized by** the steps
that the data recorded during the irradiation comprise second data which are representative for the movement of the region (40, 54) different from the target volume; and/or
that the data recorded during the irradiation comprise third data which are representative for the movement of a structure that changes the penetration depth of the particle beam; and
that a subsequent irradiation of the target volume (14) or an irradiation planning for the target volume (14) is modified based on the dose distribution determined for the region (40, 54) different from the target volume.

2. The method according to claim 1, wherein in determining the dose distribution a dose calculation is performed by means of which a dose is determined which is deposited in said region (40, 54), wherein the dose calculation is performed using an adaptation of the rescanning process to the target volume effected during the irradiation.

3. The method according to any of claims 1 or 2, wherein the subsequent irradiation is modified by a change in the irradiation planning and/or by changing an irradiation method.

4. An irradiation system for irradiating a moving target volume (14), comprising:
- an irradiation device (10) adapted to irradiate the target volume (14) in such a manner that during an irradiation the target volume (14) is irradiatable in a rescanning process in which a beam is repeatedly scanned over the target volume (14);
- a monitoring device (58) adapted to determine a dose distribution occurring in a region (40, 54) different from the target volume (14) during the irradiation of the target volume (14); and
- a movement monitoring device for recording first data during the irradiation, which are representative for the movement of the target volume,
- wherein said region (40, 54) is distinguished in that the region is subject to a different movement pattern and/or a different motion-related change in particle range than the target volume (14), and
- wherein the monitoring device is adapted to determine the dose distribution in the different region based on the data recorded by the movement monitoring device, **characterized in that**
- during the irradiation the movement monitoring device records second data which are representative for the movement of the region (40, 54) different from the target volume, and/or that during the irradiation the movement monitoring device records third data which are representative for the movement of a structure that changes the penetration depth of the particle beam,
- wherein the first and second or the first and third data permit to determine an adaptation of the rescanning process to the movement of the region different from the target volume effected during the irradiation;
- further comprising an irradiation control device (36, 44) adapted for modifying a subsequent irradiation of the target volume (14) using the dose distribution determined for the region (40, 54) different from the target volume.

5. The irradiation system according to claim 4, wherein the irradiation system comprises a detection means which determines, from the first data, an adaptation of the rescanning process to the movement of the target volume effected during the irradiation.

6. The irradiation system according to claim 5, wherein the monitoring device (58) is adapted to perform a dose calculation by means of which a dose is determined which is deposited in said region (40, 54) during the irradiation, and wherein the dose calculation is performed using the first data.

7. The irradiation system according to any of claims 4 to 6, wherein the modifying of the subsequent irradiation of the target volume (14) is accomplished by a change in the irradiation planning and/or by a change in the irradiation method.

## Revendications

1. Procédé d'irradiation d'un volume cible (14) en mouvement, dans des objets autres que le corps humain ou le corps d'animaux, ou de simulation d'une irradiation d'un volume cible en mouvement, comprenant les étapes suivantes :
- irradiation du volume cible (14), de telle façon que le volume cible (14) soit irradié selon un procédé de balayage multiple, lors duquel un faisceau balaie plusieurs fois le volume cible (14), et
- détermination d'un dépôt de dose qui est apparu dans une zone (40, 54) différente du volume cible (14), lors de l'irradiation du volume cible (14), sachant que la zone (40, 54) est soumise à un modèle de mouvement différent de celui du volume cible (14) et/ou que la zone (40, 54) est soumise à une variation de portée de particules, due au mouvement, qui est différente de celle du volume cible,
selon lequel le dépôt de dose qui apparaît pendant l'irradiation dans la zone (40, 54) différente du volume cible (14) est déterminé à l'aide de données enregistrées au cours de l'irradiation,
selon lequel les données enregistrées pendant l'irradiation comprennent des premières données qui caractérisent le mouvement du volume cible,
**caractérisé par** les étapes consistant en ce que les données enregistrées pendant l'irradiation comprennent des deuxièmes données qui caractérisent le mouvement de la zone (40, 54) différente du volume cible, et/ou que les données enregistrées pendant l'irradiation comprennent des troisièmes données qui caractérisent le mouvement d'une structure modifiant la profondeur de pénétration du faisceau de particules, et
qu'une irradiation subséquente du volume cible (14) ou un programme d'irradiation pour le volume cible (14) est modifié(e) en fonction du dépôt de dose déterminé pour la zone (40, 54) différente du volume cible.

2. Procédé selon la revendication 1, selon lequel on réalise, lors de la détermination du dépôt de dose, un calcul de dose par lequel on définit une dose qui est déposée dans la zone (40, 54), le calcul de dose étant effectué en utilisant une adaptation du procédé de balayage multiple au volume cible, qui est effectuée pendant l'irradiation.

3. Procédé selon l'une des revendications 1 ou 2, selon lequel l'irradiation subséquente est modifiée par une modification du programme d'irradiation et/ou par un changement de méthode d'irradiation.

4. Installation d'irradiation destinée à l'irradiation d'un volume cible (14) en mouvement,
- comprenant un dispositif d'irradiation (10) qui est conçu pour l'irradiation du volume cible (14), de telle sorte que lors d'une irradiation, le volume cible (14) puisse être irradié par un procédé de balayage multiple, selon lequel un faisceau balaie plusieurs fois le volume cible (14),
- comprenant un dispositif de surveillance (58) qui est conçu pour déterminer un dépôt de dose qui apparaît dans une zone (40, 54) différente du volume cible (14), lors de l'irradiation du volume cible (14), et
- comprenant un dispositif de surveillance de mouvement destiné à enregistrer des premières données pendant l'irradiation, qui caractérisent le mouvement du volume cible,
- sachant que la zone (40, 54) est **caractérisée par le fait que** la zone est soumise à un autre modèle de mouvement et/ou à une autre variation de portée de particules, due au mouvement, que le volume cible (14), et
- sachant que le dispositif de surveillance est conçu pour déterminer le dépôt de dose dans la zone différente, à l'aide des données enregistrées du dispositif de surveillance de mouvement,
**caractérisée en ce que**
- au cours de l'irradiation, le dispositif de surveillance de mouvement enregistre des deuxièmes données qui caractérisent le mouvement de la zone (40, 54) différente du volume cible, et/ou **en ce que**, au cours de l'irradiation, le dispositif de surveillance de mouvement enregistre des troisièmes données qui caractérisent le mouvement d'une structure modifiant la profondeur de pénétration du faisceau de particules,
- les premières et deuxièmes données ou les premières et troisièmes données permettant de déterminer une adaptation du procédé de balayage multiple, exécuté pendant l'irradiation, au mouvement de la zone différente du volume cible;
- comprenant en outre un dispositif de commande d'irradiation (36, 44) qui est conçu pour modifier une irradiation subséquente du volume cible (14), en utilisant le dépôt de dose déterminé pour la zone (40, 54) différente du volume cible.

5. Installation d'irradiation selon la revendication 4, sachant que l'installation d'irradiation présente un dispositif de détermination qui calcule, à partir des premières données, une adaptation du procédé de balayage multiple au mouvement du volume cible, qui est effectuée au cours de l'irradiation.

6. Installation d'irradiation selon la revendication 5, sachant que le dispositif de surveillance (58) est conçu pour effectuer un calcul de dose, avec lequel on détermine une dose qui est déposée dans la zone (40, 54) lors de l'irradiation, et sachant que le calcul de dose est effectué en utilisant les premières données.

7. Installation d'irradiation selon l'une des revendications 4 à 6, sachant que la modification de l'irradiation subséquente du volume cible (14) est effectuée par le biais d'un changement du programme d'irradiation et/ou par le changement de la méthode d'irradiation.
